# FASCICULE DE BREVET EUROPEEN

(11) **EP 3 622 991 B1**
(45) Date de publication et mention de la délivrance du brevet: **15.03.2023**
(21) Numéro de dépôt: 19194939.5
(22) Date de dépôt: 02.09.2019
(51) Int. Cl.: A61M 16/00, A61M 16/20

(54) **APPAREIL D'ASSISTANCE À LA TOUX À VALVES PNEUMATIQUES COMMANDÉES**
HILFSGERÄT BEI HUSTEN MIT GESTEUERTEN PNEUMATISCHEN VENTILEN
DEVICE FOR ASSISTING WITH COUGHS WITH CONTROLLED PNEUMATIC VALVES

(30) Priorité: 14.09.2018 FR 1871041
(43) Date de publication de la demande: 18.03.2020
(73) Titulaire: EOVE, 64000 Pau (FR)
(72) Inventeur: DEHOUR, Patrick, 64000 PAU (FR); DUCEUX, Stéphane, 64000 PAU (FR); JOURDAIN, Cédric, 64000 PAU (FR)
(74) Mandataire: Air Liquide

(56) Documents cités:
- EP-A1- 0 862 922
- WO-A1-2008/102216
- WO-A1-2017/006189
- WO-A1-2017/222913
- WO-A2-2012/158394
- WO-A2-2014/184377
- CN-U- 203 370 171
- DE-A1-102016 122 187

## Description

La présente invention concerne un appareil d'insufflation et d'exsufflation de gaz, aussi appelé appareil d'assistance à la toux, de préférence portable ou portatif, utilisable pour traiter des personnes souffrant de troubles respiratoires nécessitant une aide à l'évacuation des sécrétions pulmonaires.

Les dispositifs d'assistance à la toux comprennent généralement une turbine motorisée, aussi appelée compresseur ou micro-soufflante, tournant à vitesse fixe couplée à une électrovanne permettant d'orienter le flux d'air depuis la turbine vers le patient, c'est-à-dire en insufflation avec sortie de turbine connectée fluidiquement au patient, puis depuis le patient vers la turbine, c'est-à-dire en exsufflation avec sortie de turbine connectée à l'entrée d'air de la turbine. Des capteurs de pression et de débit associés à un contrôleur compris dans des moyens de pilotage permettent de gérer les différentes phases en fonction des réglages choisis. Un oscillateur peut également être prévu pour augmenter la capacité du dispositif à mobiliser les sécrétions pulmonaires des patients traités.

Ainsi, le document EP-A-2707069 enseigne un tel équipement d'insufflation et d'exsufflation qui comporte une turbine raccordée à une vanne de sélection, qui est elle-même raccordée à un oscillateur, qui est quant à lui raccordé à un raccord de tuyau souple. Pendant l'insufflation d'air, la vanne de sélection raccorde fluidiquement l'échappement de la turbine à l'oscillateur, provoquant une pression positive au niveau du raccord de tuyau souple. A l'inverse, pendant l'exsufflation, la vanne de sélection raccorde l'admission de la turbine à l'oscillateur, provoquant une pression négative au niveau du raccord de tuyau souple. L'oscillateur est une vanne papillon à disque tournant sur 360°. Pendant l'insufflation, le disque est prévu pour moduler progressivement le débit d'air, alors que, pendant l'exsufflation, l'oscillateur est inactif ou en mode flottement. Quand l'oscillateur est inactif, le disque est maintenu fixe pour permettre un débit d'air maximal, alors qu'en mode flottement, le disque tourne en permanence de sorte que le débit d'air alterne rapidement entre maximum et minimum.

CN-U-203370171 enseigne un appareil à toux à fonctionnement électromécanique similaire, comprenant un système oscillant coopérant avec des valves à diaphragmes.

Toutefois, les systèmes de sélection actuels posent certains problèmes.

Ainsi, le principe de maintenir la turbine à vitesse constante ne permet qu'une maitrise limitée de la pression d'air pendant les phases d'insufflation ou d'exsufflation. En particulier, lorsque l'opérateur règle une pression négative de -x mbar, cette pression n'est en réalité atteinte qu'à la fin de l'expiration active du patient et non pas au cours de la phase la plus importante pour l'efficacité du traitement.

Par ailleurs, les électrovannes utilisées dans les appareils actuels sont consommatrices d'énergie et les batteries d'alimentation de capacité limitée, ce qui limite la portabilité et/ou l'autonomie de l'appareil.

On connait par ailleurs EP-A-862922 qui propose un appareil d'assistance respiratoire avec un circuit inspiratoire et un circuit expiratoire relié à un dispositif de mise en dépression. Les deux circuits comprennent des valves pneumatiques et sont indépendants, c'est-à-dire non-reliés fluidiquement l'un à l'autre.

Au vu de cela, le problème qui se pose est dès lors de proposer un appareil d'insufflation et d'exsufflation de gaz, c'est-à-dire un appareil à toux, qui soit amélioré, en particulier pouvant non seulement opérer un basculement rapide d'une phase d'insufflation à une phase d'exsufflation, et inversement, mais aussi respecter la ou les consignes de pression et/ou préférentiellement de réduire ou maîtriser la consommation énergétique de l'appareil, et par ailleurs de réduire l'encombrement global et le poids de l'appareil.

L'invention concerne alors un appareil d'insufflation et d'exsufflation de gaz, typiquement un appareil à toux, comprenant :
- une turbine motorisée comprenant une entrée de gaz et une sortie de gaz, et
- une ligne d'insufflation raccordée fluidiquement à la sortie de gaz de la turbine,
- une ligne d'exsufflation accordée fluidiquement à l'entrée de gaz de la turbine,
- et une ligne commune de fourniture de gaz à un patient raccordée fluidiquement auxdites ligne d'insufflation et ligne d'exsufflation,
caractérisé en ce qu'il comprend en outre :
- une première et une quatrième valves pneumatiques agencées sur la ligne d'exsufflation, la première valve pneumatique contrôlant une mise en communication fluidique de la ligne d'exsufflation avec l'atmosphère et la quatrième valve pneumatique contrôlant une mise en communication fluidique de la ligne commune de fourniture de gaz avec la ligne d'exsufflation,
- une deuxième et une troisième valves pneumatiques agencées sur la ligne d'insufflation, la deuxième valve pneumatique contrôlant une mise en communication fluidique de la ligne d'insufflation avec l'atmosphère et la troisième valve pneumatique contrôlant une mise en communication fluidique de la ligne d'insufflation avec la ligne commune de fourniture de gaz,
- et des moyens de commande pneumatiques commandant pneumatiquement lesdites première, deuxième, troisième et quatrième valves pneumatiques.

Selon le cas, l'appareil de l'invention peut comprendre l'une ou plusieurs des caractéristiques techniques suivantes :
- il comprend des moyens de pilotage commandant la turbine pour délivrer du gaz pendant au moins des phases d'insufflation et d'exsufflation.
- la turbine délivre de l'air.
- la deuxième valve pneumatique comprend un orifice de mise à l'atmosphère permettant de mettre la ligne d'insufflation en communication fluidique avec l'atmosphère ambiante de sorte d'y évacuer la pression gazeuse s'exerçant dans la ligne d'insufflation.
- la première valve pneumatique comprend un orifice de liaison à l'atmosphère permettant de mettre la ligne d'exsufflation en communication fluidique avec l'atmosphère ambiante de sorte de pouvoir y prélever de l'air lorsque la turbine aspire de l'air via son entrée de gaz.
- une (chaque) phase d'insufflation a une durée comprise entre 0.5 et 10 secondes, typiquement jusqu'à 5 secondes environ.
- une (chaque) phase d'exsufflation a une durée comprise entre 0.5 et 10 secondes, typiquement jusqu'à 5 secondes environ.
- optionnellement, la (chaque) phase d'insufflation et la (chaque) phase d'exsufflation qui la suit sont elles-mêmes suivies par une phase de pause (i.e. située entre une phase d'exsufflation et la phase d'insufflation suivante) pendant laquelle on délivre une pression de pause supérieure ou égale à 0 mbar dans la ligne d'insufflation et la ligne commune, de préférence une pression de pause entre 0 et 30 mbar, par exemple de l'ordre de 10 mbar.
- pendant une (chaque) phase de pause, les moyens de pilotage commandent les électrovannes et la turbine pour délivrer une pression de pause supérieure ou égale à 0 mbar dans la ligne d'insufflation et la ligne commune, de préférence une pression de pause entre 0 et 30 mbar, préférentiellement la phase de pause a une durée de pause comprise entre 0 et 5 secondes environ.
- la phase de pause a une durée de pause comprise entre 0 et 5 secondes environ, typiquement moins de 2 secondes.
- les moyens de pilotage commandent, pendant une (chaque) phase de pause, les électrovannes et la turbine pour délivrer une pression de pause supérieure ou égale à 0 mbar dans la ligne d'insufflation et la ligne commune, de préférence une pression de pause entre 0 et 30 mbar
- les moyens de pilotage comprennent un contrôleur numérique relié électriquement à la turbine.
- les moyens de pilotage comprennent un microprocesseur, de préférence porté par une carte électronique.
- la turbine comprend un moteur électrique, c'est-à-dire fonctionnant grâce à un courant électrique.
- la turbine comprend un moteur électrique entraînant une roue à ailettes agencée dans une volute, c'est-à-dire dans le compartiment interne de la volute.
- les moyens de commande pneumatiques commandent pneumatiquement lesdites première, deuxième, troisième et quatrième valves pneumatiques en fonction des phases d'insufflation et d'exsufflation de gaz de la turbine, et éventuellement de la phase de pause et/ou de la phase transitoire.
- pendant une (chaque) phase d'insufflation de gaz et/ou une (chaque) phase de pause éventuelle, les moyens de commande pneumatiques commandent pneumatiquement :
- la deuxième valve pneumatique pour empêcher toute communication fluidique entre la ligne d'insufflation et l'atmosphère,
- la troisième valve pneumatique pour opérer une mise en communication fluidique de la ligne d'insufflation avec la ligne commune de fourniture de gaz,
- la première valve pneumatique pour opérer une mise en communication fluidique de la ligne d'exsufflation avec l'atmosphère, et
- la quatrième valve pneumatique pour empêcher toute communication fluidique entre la ligne commune de fourniture de gaz et la ligne d'exsufflation.
- pendant une phase d'insufflation et/ou une phase de pause éventuelle, la turbine délivre de l'air sous pression (i.e. > à la pression atmosphérique) dans la ligne d'insufflation raccordée fluidiquement à la sortie de gaz de la turbine, c'est-à-dire que la turbine génère une pression positive (P+) dans ladite ligne d'insufflation. L'air sous pression y chemine en direction de la ligne commune de fourniture de gaz et donc du patient.
- pendant une (chaque) phase d'exsufflation de gaz, les moyens de commande pneumatiques commandent pneumatiquement :
- la deuxième valve pneumatique pour opérer une mise en communication fluidique de la ligne d'insufflation avec l'atmosphère,
- la troisième valve pneumatique pour empêcher toute communication fluidique entre la ligne d'insufflation et la ligne commune de fourniture de gaz,
- la première valve pneumatique pour empêcher toute communication fluidique entre la ligne d'exsufflation et l'atmosphère, et
- la quatrième valve pneumatique pour opérer une mise en communication fluidique de la ligne commune de fourniture de gaz avec la ligne d'exsufflation.
- pendant une (chaque) phase d'insufflation, la turbine génère une pression positive (P+) comprise entre 5 et 70 mbar (pression relative par rapport à la pression atmosphérique).
- pendant une (chaque) phase de pause éventuelle, la turbine génère une pression positive (P+) comprise entre 0 et 30 mbar (pression relative par rapport à la pression atmosphérique).
- pendant une (chaque) phase d'exsufflation, la turbine génère une pression négative (P-) du côté de l'entrée de gaz de la turbine, c'est-à-dire une dépression (i.e. une pression inférieure ou égale à la pression atmosphérique), dans la ligne d'exsufflation et dans la ligne commune de fourniture de gaz qui est fluidiquement raccordée à la ligne d'exsufflation. Ceci permet de mettre les voies respiratoires du patient en dépression. L'air aspiré est ensuite délivré dans la ligne d'insufflation, puis évacué vers l'atmosphère ambiante, via l'orifice de mise à l'atmosphère de la deuxième valve pneumatique.
- pendant une phase (chaque) d'exsufflation, la turbine génère une pression négative (P-) comprise entre 0 et -70 mbar (pression relative par rapport à la pression atmosphérique).
- la turbine est commandée pour atteindre une vitesse de rotation maximale de 75000 tours/minute.
- optionnellement, la phase d'insufflation et la phase d'exsufflation sont espacées l'une de l'autre par une phase transitoire permettant de préparer l'état des valves (i.e. ouverture ou fermeture complète...) et des circuits de gaz (e.g. pressions dans les lignes d'insufflation et d'exsufflation...) pour que le démarrage de la phase d'exsufflation suivante s'effectue à un débit d'exsufflation donné, préférentiellement un débit d'exsufflation maximal.
- chaque phase transitoire débute en fin de phase d'insufflation, c'est-à-dire qu'elle est opérée à la fin une phase d'insufflation et avant la phase d'exsufflation suivante.
- une (chaque) phase transitoire a une durée comprise entre 50 et 500 ms environ.
- pendant au moins une (chaque) phase transitoire, les moyens de commande pneumatiques commandent pneumatiquement :
- la deuxième valve pneumatique pour empêcher toute mise en communication fluidique de la ligne d'insufflation avec l'atmosphère,
- la troisième valve pneumatique pour empêcher toute communication fluidique entre la ligne d'insufflation et la ligne commune de fourniture de gaz,
- la première valve pneumatique pour empêcher toute communication fluidique entre la ligne d'exsufflation et l'atmosphère, et
- la quatrième valve pneumatique pour empêcher toute mise en communication fluidique de la ligne commune de fourniture de gaz avec la ligne d'exsufflation.
- les moyens de commande pneumatiques comprennent des première et seconde électrovannes.
- les première et seconde électrovannes comprennent des électrovannes pneumatiques.
- les moyens de commande pneumatiques comprennent des première et seconde électrovannes de type « tout ou rien » et/ou à 3 voies.
- les première et seconde électrovannes sont pilotées électriquement par les moyens de pilotage.
- les première et seconde électrovannes sont connectées fluidiquement à une source de pression positive, i.e. une source de gaz sous pression.
- la source de pression positive connectée fluidiquement aux première et seconde électrovannes est, selon le cas, la turbine ou une source de pression additionnelle de type (micro)pompe, (micro)compresseur ou analogue.
- avantageusement, la source de pression positive est une source de pression additionnelle de type (micro)pompe, (micro)compresseur ou analogue.
- la source de pression additionnelle est connectée fluidiquement aux première et seconde électrovannes, via au moins une ligne d'amenée de pression positive.
- les première et seconde électrovannes sont par ailleurs connectées fluidiquement à une source de pression dite négative, appelée source de pression négative, via au moins une ligne d'amenée de pression négative.
- les première et seconde électrovannes sont par ailleurs connectées fluidiquement à une source de pression négative qui est la ligne d'exsufflation ou l'atmosphère ambiante.
- les première et seconde électrovannes pneumatiques sont des électrovannes dites « miniatures » consommant moins de 10 Watt, de préférence de l'ordre de 5 Watt ou moins, en pic de consommation instantanée.
- les première et seconde électrovannes pneumatiques sont de type à 3 voies comprenant deux entrées pneumatiques et une sortie pneumatique.
- les première et seconde électrovannes comprennent chacune :
- .une première voie (avec une première entrée pneumatique) reliée à la source de pression positive,
- .une deuxième voie (avec une seconde entrée pneumatique) reliée à la source de pression négative, et
- .une troisième voie (avec sortie pneumatique) reliée à une ou plusieurs valves pneumatiques, de préférence via une ligne de commande pneumatique.
- la première électrovanne commande pneumatiquement les deuxième et quatrième valves pneumatiques.
- la seconde électrovanne commande pneumatiquement les première et troisième valves pneumatiques.
- la première électrovanne et la seconde électrovanne commandent pneumatiquement les valves pneumatiques, via leur troisième voie comprenant leur sortie pneumatique.
- les moyens de pilotage commandent électriquement les première et seconde électrovannes de manière à raccorder fluidiquement :
- soit la première voie à la troisième voie de manière à fournir une pression de commande positive,
- soit la deuxième voie à la troisième voie de manière à fournir une pression de commande négative ou nulle.
- la deuxième voie est normalement (i.e. repos ou par défaut par exemple) reliée la troisième voie de chacune des première et seconde électrovannes, c'est-à-dire en l'absence de commande par les moyens de pilotage.
- les moyens de pilotage commandent électriquement les première et seconde électrovannes en fonction des phases d'insufflation et d'exsufflation, et optionnellement en fonction aussi de la phase de pause et/ou de la phase transitoire.
- il comprend une carcasse externe rigide dans laquelle sont agencés au moins la turbine motorisée, la ligne d'insufflation, la ligne d'exsufflation, la ligne commune, les première, deuxième, troisième et quatrième valves pneumatiques, et les moyens de commande pneumatiques commandant pneumatiquement les valves pneumatiques, en particulier les électrovannes pneumatiques.
- il comprend une IHM ou interface homme-machine permettant à un utilisateur, par exemple un personnel soignant ou autre, de rentrer une ou des valeurs de consigne, notamment des valeurs de pression haute et basse, de temps des différentes phases...
- l'IHM comprend un écran digital à touches tactiles, de préférence à affichage en couleurs.
- il comprend des moyens d'alimentation en courant électrique alimentant en courant électrique au moins la turbine motorisée, l'IHM et les moyens de pilotage et, éventuellement, la pompe ou le compresseur.
- les moyens d'alimentation en courant électrique comprennent au moins une batterie ou pile, de préférence rechargeable, et/ou une prise électrique et un cordon électrique de raccordement au secteur (e.g. 110/230 V), et éventuellement un transformateur de courant.
- la ligne commune de fourniture de gaz est reliée fluidiquement au patient via une interface respiratoire, tel un masque respiratoire, par exemple un masque bucco-nasal, ou une sonde de trachéotomie ou encore un embout buccal.
- la ligne commune de fourniture de gaz est reliée fluidiquement à l'interface respiratoire par l'intermédiaire un tuyau flexible ou analogue.
- l'appareil comprend une station périphérique venant entourer et loger un module de ventilation central extractible, c'est-à-dire deux parties venant s'imbriquer de manière désolidarisable/détachable, l'une dans l'autre.
- le module de ventilation central vient s'emboiter/s'insérer de manière extractible/détachable dans la station périphérique.
- le module de ventilation central comprend la carcasse externe rigide dans laquelle sont agencés au moins la turbine motorisée, la ligne d'insufflation, la ligne d'exsufflation, la ligne commune, les première, deuxième, troisième et quatrième valves pneumatiques, les électrovannes pneumatiques et les moyens de pilotage...
- l'IHM est agencée, de manière détachable ou non, sur la station périphérique.

L'invention va maintenant être mieux comprise grâce à la description détaillée suivante, faite à titre illustratif mais non limitatif, en référence aux figures annexées parmi lesquelles :
- [Fig. 1] est un schéma d'un mode de réalisation de l'architecture interne d'un appareil d'insufflation et d'exsufflation de gaz selon l'invention,
- [Fig. 2] est un chronogramme obtenu lors du fonctionnement de l'appareil de la [Fig. 1],
- [Fig. 3] et [Fig. 4] illustrent un mode de réalisation d'un appareil d'insufflation et d'exsufflation de gaz selon l'invention,
- [Fig. 5] schématise le fonctionnement des électrovannes et des valves pneumatiques de l'appareil de la [Fig. 1].

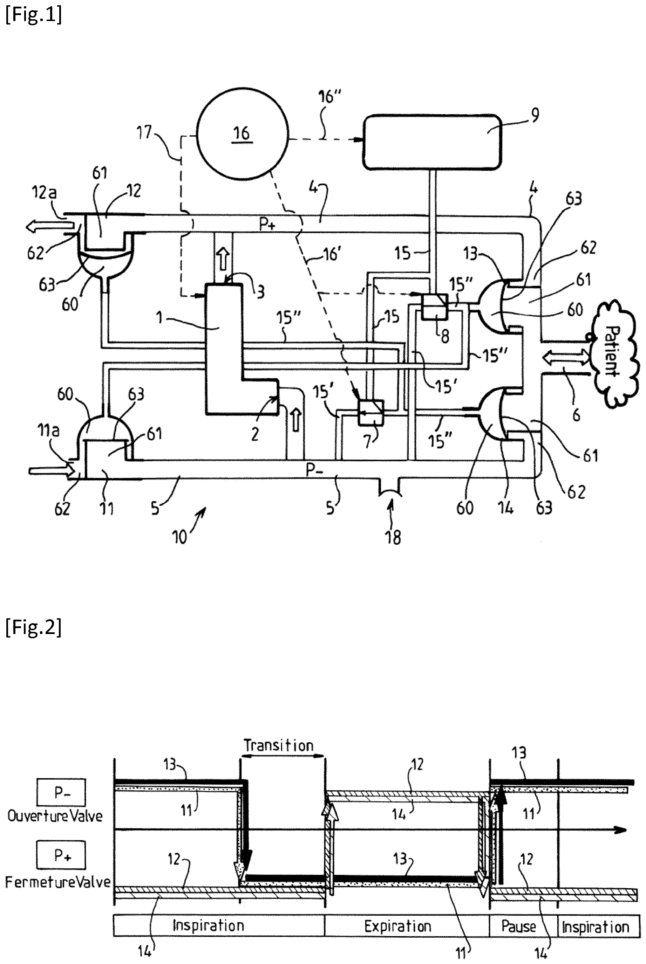

[Fig. 1] schématise l'architecture interne d'un appareil d'insufflation et d'exsufflation de gaz 10 selon l'invention, aussi appelé appareil d'assistance à la toux, utilisable pour traiter des personnes souffrant de troubles respiratoires nécessitant une aide à l'évacuation des sécrétions pulmonaires. Avantageusement, cet appareil 10 est portable ou portatif (i.e. utilisable pendant une déambulation), de préférence portable.

Il comprend une turbine 1 motorisée, aussi appelé compresseur ou micro-soufflante, comprenant une entrée de gaz 2 par laquelle l'air est aspiré et pénètre dans la turbine 1 et une sortie de gaz 3 par laquelle l'air, de préférence à pression positive, est expulsé et ressort de la turbine 1. La turbine 1 comprend classiquement un moteur électrique entraînant une roue à ailettes agencée dans le compartiment interne de la volute et servant à délivrer de l'air sous pression (> 1 bar).

Il est également prévu des moyens de pilotage 16 commandant la turbine 1 pour délivrer du gaz pendant au moins des phases d'insufflation et d'exsufflation, comme détaillé ci-après.

Optionnellement, la/chaque phase d'insufflation et la/chaque phase d'exsufflation peuvent être suivies par une phase de pause qui est située entre la phase d'exsufflation et la phase d'insufflation suivante. La phase de pause a une durée de pause comprise entre 0 et 5 secondes environ, typiquement moins de 2 secondes.

Pendant cette (chaque) phase de pause, la turbine 1 est commandée par les moyens de pilotage 16 pour délivrer une pression gazeuse de pause supérieure ou égale à 0 mbar dans la ligne d'insufflation 4 et la ligne commune 6, typiquement une pression de pause entre 0 et 30 mbar, par exemple de l'ordre de 10 mbar.

Plus précisément, les moyens de pilotage agissent, pendant la phase de pause, sur les électrovannes 11-14, via les moyens de commande pneumatique, c'est-à-dire les électrovannes 7, 8, comme expliqué ci-après, et la turbine 1 pour délivrer la pression de pause désirée.

De préférence, les moyens de pilotage 16 comprennent un contrôleur numérique relié électriquement 17 à la turbine 1. Le contrôleur numérique des moyens de pilotage 16 peut comprendre par exemple une carte électronique à microcontrôleur mettant en oeuvre un ou plusieurs algorithmes servant à piloter la turbine 1 et des moyens de mémorisation, telle une mémoire flash ou autre.

Pendant le fonctionnement de l'appareil 1, la turbine 1 alimente en air, une ligne d'insufflation 4 qui est raccordée fluidiquement à la sortie de gaz 3 de la turbine 1 et permet d'acheminer l'air expulsé par la turbine 1. Par ailleurs, une ligne d'exsufflation 5 est quant à elle raccordée fluidiquement à l'entrée de gaz 2 de la turbine 1 et permet notamment d'y acheminer l'air aspiré par la turbine 1. Les lignes d'insufflation 4 et d'exsufflation 5 sont par exemple des conduits de gaz, des passages de gaz ou analogues, agencés dans l'appareil 10.

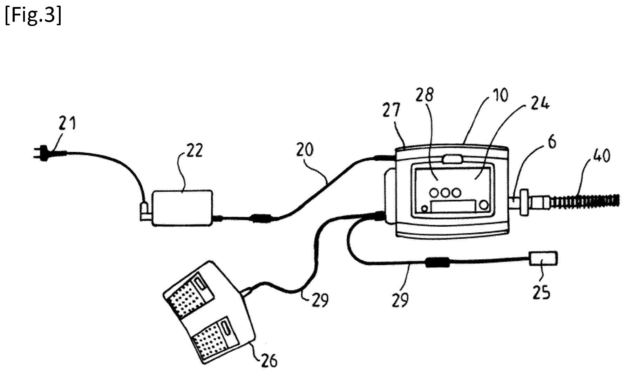

Les lignes d'insufflation 4 et d'exsufflation 5 sont par ailleurs raccordées fluidiquement à une ligne commune 6 de fourniture de gaz, tel un conduit de gaz ou analogue, qui est reliée fluidiquement à un patient, par exemple via un tuyau flexible 40 (cf. [Fig. 3]) relié fluidiquement à une interface respiratoire (e.g. masque respiratoire, sonde trachéale etc...), de sorte d'alimenter les voies respiratoires du patient, notamment ses poumons, en air sous pression pendant les phases d'insufflation et, à l'inverse, d'en extraire le gaz pendant les phases d'exsufflation.

Les lignes d'insufflation 4 et d'exsufflation 5 comprennent plusieurs valves pneumatiques 11-14 permettant de contrôler les flux gazeux pendant les phases d'insufflation et d'exsufflation successives, et optionnellement pendant les phases de pause et/ou transitoires.

Plus précisément, une première 11 et une quatrième 14 valves pneumatiques sont agencées sur la ligne d'exsufflation 5. La première valve pneumatique 11 contrôle la mise en communication fluidique de la ligne d'exsufflation 5 avec l'atmosphère ambiante, via un orifice d'entrée 11a, pour permettre à de l'air atmosphérique de d'y pénétrer et d'alimenter la turbine 1, pendant les phases d'insufflation, comme expliqué ci-après, alors que la quatrième valve pneumatique 14 contrôle la mise en communication fluidique de la ligne commune 6 de fourniture de gaz avec la ligne d'exsufflation 5, pendant les phases d'exsufflation, comme expliqué ci-après.

Par ailleurs, une deuxième 12 et une troisième 13 valves pneumatiques sont agencées sur la ligne d'insufflation 4. Dans ce cas, la deuxième valve pneumatique 12 contrôle la mise en communication fluidique de la ligne d'insufflation 4 avec l'atmosphère, via un orifice d'évacuation de gaz 12a pour évacuer le gaz sous pression, pendant les phases d'exsufflation, comme expliqué ci-après, alors que la troisième valve pneumatique 13 contrôle la mise en communication fluidique de la ligne d'insufflation 4 avec la ligne commune 6 de fourniture de gaz, pour alimenter le patient en air, pendant les phases d'insufflation, comme expliqué ci-après.

Ces première, deuxième, troisième et quatrième valves pneumatiques 11-14 sont elles-mêmes pilotées pneumatiquement par des moyens de commande pneumatiques 7, 8 comprenant des première et seconde électrovannes pneumatiques 7, 8. Les première et seconde électrovannes 7, 8 sont des électrovannes miniatures à faible consommation de pic (i.e. < 10 Watt).

Les première et seconde électrovannes pneumatiques 7, 8 sont pilotées électriquement, via une ou plusieurs liaisons électriques 16', par les moyens de pilotage 16 de l'appareil 10, en fonction de la phase d'insufflation ou d'exsufflation à opérer, ou des éventuelles phases de pause et/ou transitoire.

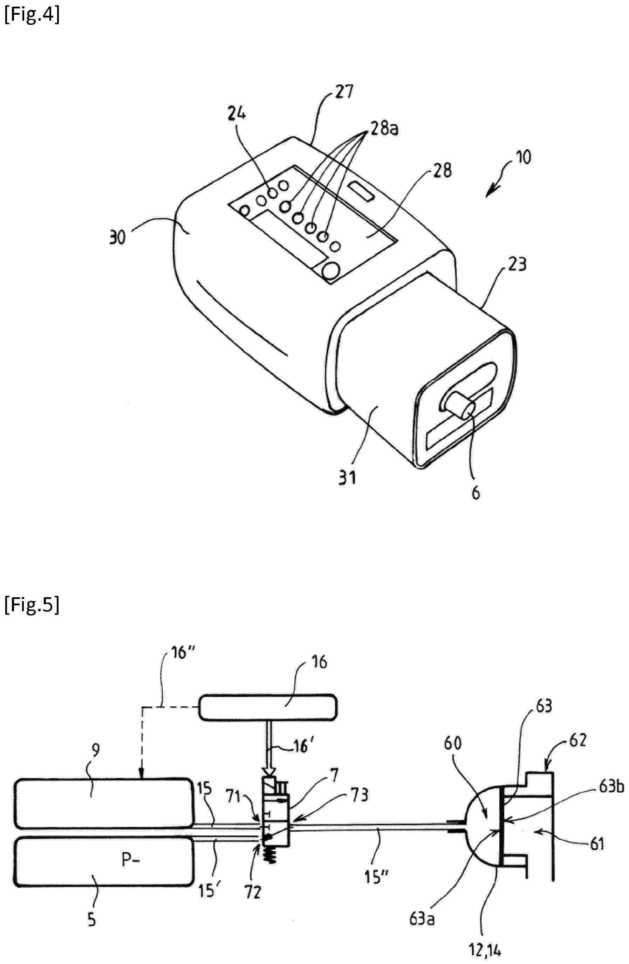

Comme illustré en [Fig. 1] et [Fig. 5], les première et seconde électrovannes pneumatiques 7, 8 sont par ailleurs reliées pneumatiquement à au moins une ligne de pilotage pneumatique 15, c'est-à-dire au moins une ligne d'amenée de pression positive, par exemple une conduite de gaz qui se ramifie ou plusieurs conduites, reliant ces première et seconde électrovannes pneumatiques 7, 8 à une source de pression positive 9, i.e. une source de gaz sous pression.

Cette source de pression positive 9 alimentant les première et seconde électrovannes 7, 8 en gaz sous pression peut être, selon le mode de réalisation considéré, soit la turbine 1, soit une source de pression additionnelle 9 comme une pompe (aussi appelée micro-pompe), un compresseur (aussi appelée micro-compresseur) ou analogue alimentant fluidiquement la ligne de pilotage pneumatique 15.

Préférentiellement, comme représenté en [Fig. 1], la source de pression positive 9 est une (micro)pompe ou un (micro)compresseur délivrant du gaz sous pression. Elle est par ailleurs pilotée par les moyens de pilotage 16, typiquement par le microcontrôleur, via une (ou plusieurs) liaison électrique 16". L'usage d'une pompe ou d'un compresseur additionnel permet d'obtenir notamment plus d'efficacité ou de réactivité pendant les phases de transitions éventuelles, c'est-à-dire pendant les périodes de temps séparant les phases d'insufflation et d'exsufflation.

En outre, les première et seconde électrovannes 7, 8 sont aussi connectées fluidiquement à une source de pression dite « négative », appelée simplement source de pression négative, via au moins une ligne d'amenée de pression négative 15', à savoir une conduite qui se ramifie ou plusieurs conduites par exemple. Cette source de pression négative peut être soit la ligne d'exsufflation 5 où règne une pression négative (P-) pendant les phases d'exsufflation, soit l'atmosphère ambiante (i.e. la pression atmosphérique).

La [Fig. 5] permet de mieux comprendre le fonctionnement des électrovannes 7, 8 et la manière dont elles commandent pneumatiquement les vannes pneumatiques 11-14 de l'appareil de la [Fig. 1]. Seule la première électrovanne pneumatique 7 est représentée sur la [Fig. 5]. Toutefois, les explications données ci-après concernent indifféremment la première électrovanne pneumatique 7 et la seconde électrovanne pneumatique 8 étant donné que le fonctionnement et l'architecture de ces deux électrovannes pneumatiques sont exactement les mêmes.

La première électrovanne pneumatique 7 est de type à 3 voies, c'est-à-dire qu'elle comprend deux entrées pneumatiques 71, 72 et une sortie pneumatique 73. Plus précisément :
- la première voie comprenant la première entrée pneumatique 71 est reliée à la source de pression positive 9, via la ligne de pilotage pneumatique 15,
- la deuxième voie comprenant la seconde entrée pneumatique 72 est reliée à la source de pression négative, par exemple à la ligne d'exsufflation 5, via la ligne d'amenée de pression négative 15', et
- la troisième voie comprenant la sortie pneumatique 73 est reliée aux valves pneumatiques 12, 14, de préférence via une (ou des) ligne de commande pneumatique 15".

En fonctionnement, les moyens de pilotage 16 commandent électriquement la première électrovanne 7, en fonction des phases d'insufflation et d'exsufflation de manière à raccorder fluidiquement :
- soit la première voie (avec entrée 71) à la troisième voie (avec sortie 73) de manière à fournir une pression de commande positive aux deuxième et quatrième valves pneumatiques 12, 14,
- soit la deuxième voie (avec entrée 72) à la troisième voie (avec sortie 73) de manière à fournir une pression de commande négative aux deuxième et quatrième valves pneumatiques 12, 14.

La deuxième voie (avec entrée 72) est normalement reliée la troisième voie 73 de la première électrovanne 7, c'est-à-dire en l'absence de commande par les moyens de pilotage 16 (i.e. en repos ou par défaut par exemple).

Avantageusement, les valves pneumatiques 11-14 sont des valves à membrane. Comme illustré en [Fig. 1] et [Fig. 5], chaque valve pneumatique 11-14 comprend :
- une première cavité interne 60, appelé cavité de commande, en communication fluidique avec la troisième voie d'une des électrovannes pneumatiques 7, 8, ladite première cavité interne 60 étant soumise à la pression ou dépression provenant de la ligne de commande pneumatique 15",
- une deuxième cavité interne 61 et une troisième cavité interne 62 séparée l'une de l'autre par une membrane souple déformable 63, ladite membrane souple déformable 63 séparant en outre les deuxième 61 et troisième 62 cavités internes de la première cavité interne 60.

Lorsqu'une pression gazeuse est amenée dans la première cavité interne 60 par la ligne de commande pneumatique 15", cette pression va s'exercer sur la surface avant 63a de la membrane 63 qui va alors créer, via sa surface arrière 63b, une étanchéité gazeuse entre la deuxième 61 et la troisième 62 cavité interne en empêchant tout passage de gaz de la deuxième 61 à la troisième cavité interne 62.

A l'inverse, lorsqu'une dépression gazeuse est amenée dans la première cavité interne 60 par la ligne de commande pneumatique 15", plus aucune pression ne va alors s'exercer sur la surface avant 63a de la membrane 63 qui va alors libérer le passage, du côté de sa surface arrière 63b, entre la deuxième 61 et la troisième 62 cavité interne en autorisant alors le passage de gaz de la deuxième 61 à la troisième cavité interne 62.

Ces passages ou interruptions de passage de gaz entre les deuxième 61 et troisième 62 cavités internes des valves 11-14 va permettre de contrôler les circulations de gaz dans l'appareil 10, comme expliqué ci-après, en fonction de la phase d'insufflation ou d'exsufflation à opérer.

Pendant le fonctionnement de l'appareil 10, la première électrovanne 7 commande les deuxième et quatrième valves pneumatiques 12, 14, alors que la seconde électrovanne 8 commande les première et troisième valves pneumatiques 11, 13, et ce, en fonction des phases successives d'insufflation et d'exsufflation de gaz, voire de celles de pause et/ou transitoires, en réponse aux signaux de commande provenant des moyens de pilotage 16.

Pendant chaque phase d'insufflation, les moyens de commande pneumatiques commandent pneumatiquement la deuxième valve pneumatique 12 pour empêcher toute communication fluidique entre la ligne d'insufflation 4 et l'atmosphère, via l'orifice d'évacuation 12a situé dans la troisième cavité interne 62 de la deuxième valve pneumatique 12 ; la troisième valve pneumatique 13 pour opérer une mise en communication fluidique de la ligne d'insufflation 4 avec la ligne commune 6 de fourniture de gaz, via les deuxième et troisième cavités 61, 62 de la troisième valve pneumatique 13; la première valve pneumatique 11 pour opérer une mise en communication fluidique de la ligne d'exsufflation 5 avec l'atmosphère, via l'orifice d'entrée 11a situé dans la troisième cavité interne 62 de la première valve pneumatique 11; et la quatrième valve pneumatique 14 pour empêcher toute communication fluidique entre la ligne commune 6 de fourniture de gaz et la ligne d'exsufflation 5.

En d'autres termes, pendant chaque phase d'insufflation, la turbine 1 délivre de l'air sous pression (> 1 bar abs) dans la ligne d'insufflation 4 raccordée fluidiquement à la sortie 3 de gaz de la turbine 1, c'est-à-dire que la turbine 1 génère une pression positive (P+) dans ladite ligne d'insufflation 4. L'air sous pression y chemine en direction de la ligne commune 6 de fourniture de gaz et donc du patient, auquel l'air est distribué par la conduite flexible 40 (cf. [Fig. 3]) et une interface respiratoire reliée fluidiquement à ladite conduite flexible 40. Dans ce cas, la turbine 1 génère une pression positive (P+) comprise entre par exemple 0 et 70 mbar.

A l'inverse, pendant une phase d'exsufflation de gaz, les moyens de commande pneumatiques 7, 8, 15 commandent pneumatiquement la deuxième valve pneumatique 12 pour opérer une mise en communication fluidique de la ligne d'insufflation 4 avec l'atmosphère, via l'orifice d'évacuation 12a ; la troisième valve pneumatique 13 pour empêcher toute communication fluidique entre la ligne d'insufflation 4 et la ligne commune 6 de fourniture de gaz ; la première valve pneumatique 11 pour empêcher toute communication fluidique entre la ligne d'exsufflation 5 et l'atmosphère, via l'orifice d'entrée 11a; et la quatrième valve pneumatique 14 pour opérer une mise en communication fluidique de la ligne commune 6 de fourniture de gaz avec la ligne d'exsufflation 5, via les deuxième et troisième cavités 61, 62 de la quatrième valve pneumatique 14.

Dit autrement, pendant une phase d'exsufflation, la turbine 1 génère une pression négative (P-) du côté de l'entrée 2 de gaz de la turbine 1, c'est-à-dire un vide, dans la ligne d'exsufflation 5 et dans la ligne commune 6 de fourniture de gaz qui est fluidiquement raccordée à la ligne d'exsufflation 5. Ceci permet de mettre le tractus respiratoire du patient en dépression. L'air aspiré est ensuite délivré dans la ligne d'insufflation 5, puis évacué vers l'atmosphère ambiante, via l'orifice de mise à l'atmosphère 12a de la deuxième valve pneumatique 12. Dans ce cas, la turbine 1 génère une pression négative (P-) comprise entre par exemple 0 et -70 mbar.

Pendant les phases d'insufflation et d'exsufflation, la turbine 1 est commandée par les moyens de pilotage 16. Typiquement, la vitesse de rotation maximale de la turbine peut atteindre environ 75 000 tours/minute.

De façon générale, l'invention repose donc sur une combinaison de valves pneumatiques 11-14 permettant un basculement rapide de la sortie patient, c'est-à-dire de la ligne commune 6, entre l'entrée 2 et la sortie 3 de la turbine 1.

Ces valves pneumatiques 11-14 sont commandées pneumatiquement par les électrovannes pneumatiques 7, 8 qui sont de type « tout ou rien », lesquelles sont elles-mêmes pilotées électriquement par les moyens de pilotage 16 et reliées fluidiquement à une pompe ou un compresseur additionnel 9 délivre du gaz sous pression.

Le chronogramme de la [Fig. 2] schématise le pilotage, en fonction du temps (T), par exemple exprimé en secondes, des électrovannes pneumatiques 7, 8 agissant sur les valves pneumatiques 11-14 pendant les phases d'insufflation et d'exsufflation, c'est-à-dire lorsque le patient inspire puis expire du gaz, et pendant une phase transitoire, aussi appelée phase de transition, et une phase de pause.

Les électrovannes 7, 8 pilotent, respectivement, les valves pneumatiques 12, 14 et les valves 11, 13, respectivement, en pression positive ou, selon le cas, négative, comme expliqué ci-avant.

Plus précisément, durant la phase d'insufflation de gaz, c'est-à-dire pendant l'inspiration du patient, la turbine 1 génère une pression positive P+ en sortie 3 de turbine 1, qui est envoyée au patient via la troisième valve 13. Pour pouvoir délivrer du débit, la turbine 1 aspire l'air ambiant au travers de la première valve 11. Les première et troisième valves 11, 13 sont donc pilotées pour être ouverte « O », alors que les deuxième et quatrième valves 12 et 14 sont pilotées pour être maintenues fermées « F » pour assurer la circulation de l'air uniquement dans le sens voulu.

Ensuite, durant la phase d'exsufflation, c'est-à-dire pendant l'expiration du patient, la turbine 1 génère une pression négative P- (i.e. dépression) en entrée 2 de turbine 1 qui est transmise au patient, via la ligne d'exsufflation 5 et la quatrième valve 14. Pour pouvoir aspirer du débit, donc mettre la ligne d'exsufflation 5 et la quatrième valve 14 en dépression, la turbine 1 doit pouvoir expulser de la pression, donc du gaz, au travers de la deuxième valve 12 vers l'atmosphère ambiante. Les première et troisième valves 11 et 13 sont donc pilotées pour être fermées de manière à assurer la circulation de l'air uniquement dans le sens voulu.

Avantageusement, les deux phases de fonctionnement, à savoir la phase d'insufflation (Inspiration) et la phase d'exsufflation (Expiration), peuvent être séparées l'une de l'autre par une phase transitoire (Transition) optionnelle, comme illustré en Figure 2, durant laquelle toutes les valves 11-14 sont fermées. Elle peut durer jusqu'à environ 5 secondes. Cette phase de transition permet de préparer la phase d'exsufflation suivante (i.e. phase d'expiration) en isolant le patient du circuit de la turbine 1 et en isolant par ailleurs le circuit de la turbine 1 de l'atmosphère ambiante, ce qui engendre une pression négative en entrée 2 de turbine permettant d'obtenir une transition rapide entre pression positive et pression négative au niveau du patient dès le début de la phase expiratoire, i.e. d'exsufflation, suivante, avec création d'un pic de débit.

En outre, selon un mode de fonctionnement particulier, une autre phase dite de pause (Pause), comme illustrée en [Fig. 2], se situant après une phase d'exsufflation (Expiration) et avant la phase d'insufflation suivante (inspiration), peut être opérée pour permettre au patient de mieux préparer la phase d'insufflation suivante.

Préférentiellement, la (chaque) phase de pause a une durée de pause comprise entre 0 et 5 secondes environ.

Durant cette (chaque) phase de pause, les valves 7, 8 sont configurées comme dans la phase d'insufflation et la turbine 1 génère une pression de pause qui peut être légèrement positive en direction du patient ou alors qui peut être égale à la pression atmosphérique.

En d'autres termes, les moyens de pilotage 16 pilotent électriquement les électrovannes 7, 8 et la turbine 1 pour délivrer une pression de pause supérieure ou égale à 0 mbar dans la ligne d'insufflation 4 et la ligne commune 6, typiquement une pression de pause entre 0 et 30 mbar.

En outre, on peut aussi prévoir un clapet de décharge 18, par exemple un clapet anti-retour, entre dans la ligne d'exsufflation 5, c'est-à-dire en amont de l'entrée 2 de la turbine, afin d'éliminer des pressions positives transitoires éventuelles pouvant se produire au sein de la ligne d'exsufflation 5, et ainsi s'assurer que les valves s'ouvrent correctement.

Selon une autre variante de l'invention, on peut aussi agencer dans l'appareil, une électrovanne supplémentaire pour piloter les valves 11 à 14, c'est-à-dire en plus des électrovannes 7, 8.

[Fig. 3] et [Fig. 4] représentent une vue générale d'un mode de réalisation d'un appareil d'insufflation et d'exsufflation de gaz 10 selon l'invention, aussi appelé appareil d'assistance à la toux, utilisable pour traiter des personnes souffrant de troubles respiratoires nécessitant une aide à l'évacuation des sécrétions pulmonaires.

Dans ce mode de réalisation, l'appareil 10 comprend deux parties 30, 31 venant s'imbriquer l'une dans l'autre, comme illustré en [Fig. 4], à savoir une station périphérique 30 venant entourer et abriter un module de ventilation central 31 extractible.

Plus précisément, le module de ventilation central 31 vient s'emboiter/s'insérer de manière extractible/détachable dans la station périphérique 30 comme montré en Figure 4 où le module de ventilation central 31 est à moitié inséré dans la station périphérique 30.

Les éléments de l'appareil 10 montrés sur la [Fig. 1] peuvent être agencés dans la carcasse rigide 23 du module de ventilation central 31 de l'appareil 10, par exemple une carcasse en polymère ou analogue.

Une interface homme-machine ou IHM 24 est agencée ici sur la station périphérique 30, par exemple l'IHM 24 est portée par la coque rigide 27 de la station 30, par exemple une coque en polymère ou analogue. L'IHM 24 permet à l'utilisateur de rentrer des valeurs de consigne dans l'appareil 10, notamment des valeurs de pression haute et basse... L'IHM 24 peut comprendre par exemple un écran digital 28, de préférence à affichage en couleurs, à touches tactiles 28a.

Comme visible sur la [Fig. 3], il est aussi prévu des moyens d'alimentation en courant électrique alimentant en courant électrique les différents composants de l'appareil 10 nécessitant de l'être, à savoir notamment la turbine 1 motorisée, les moyens de pilotage 16, l'IHM 24.... et éventuellement le micro-compresseur 9 ou d'autres composants.

Les moyens d'alimentation en courant électrique comprennent par exemple une (ou plusieurs) batterie ou pile, de préférence rechargeable, et/ou une prise électrique 21 et un cordon électrique 20 de raccordement au secteur, e.g. 110/230 V, avec ou sans transformateur 22 de courant, comme montré sur la Figure 3.

Par ailleurs, l'appareil 10 peut aussi être raccordé, via un ou des câbles de liaison 29 adaptés, à d'autres accessoires, par exemple un oxymètre 25 servant à mesurer la SpO₂ du patient, un dispositif de contrôle à pédales 26 servant à contrôler « manuellement » les phases d'insufflation et d'exsufflation, et éventuellement de pause.

Dans le mode de réalisation des [Fig. 3] et [Fig. 4], l'appareil 10 comprend deux modules 30, 31 venant s'insérer l'un dans l'autre ; toutefois, il est entendu que l'appareil selon l'invention pourrait ne comprendre qu'un seul module.

D'une manière générale, un appareil d'insufflation et d'exsufflation de gaz selon la présente invention peut être utilisé chez des patients incapables de gérer seuls leurs sécrétions de sorte de leur fournir des insufflations et exsuffiations de gaz (i.e. d'air), que ces patients soient des patients adultes ou pédiatriques. Il peut être utilisé à domicile ou à l'hôpital, avec des interfaces respiratoires invasives (e.g. sondes trachéales) ou non-invasives (e.g. masques).

## Revendications

1. Appareil d'insufflation et d'exsufflation de gaz (10) comprenant :
- une turbine (1) motorisée comprenant une entrée de gaz (2) et une sortie de gaz (3), et
- une ligne d'insufflation (4) raccordée fluidiquement à la sortie de gaz (3) de la turbine (1),
- une ligne d'exsufflation (5) raccordée fluidiquement à l'entrée de gaz (2) de la turbine (1), et
- une ligne commune (6) de fourniture de gaz à un patient raccordée fluidiquement auxdites ligne d'insufflation (4) et ligne d'exsufflation (5),
**caractérisé en ce qu'**il comprend en outre :
- une première (11) et une quatrième (14) valves pneumatiques agencées sur la ligne d'exsufflation (5), la première valve pneumatique (11) contrôlant une mise en communication fluidique de la ligne d'exsufflation (5) avec l'atmosphère et la quatrième valve pneumatique (14) contrôlant une mise en communication fluidique de la ligne commune (6) de fourniture de gaz avec la ligne d'exsufflation (5),
- une deuxième (12) et une troisième (13) valves pneumatiques agencées sur la ligne d'insufflation (4), la deuxième valve pneumatique (12) contrôlant une mise en communication fluidique de la ligne d'insufflation (4) avec l'atmosphère et la troisième valve pneumatique (13) contrôlant une mise en communication fluidique de la ligne d'insufflation (4) avec la ligne commune (6) de fourniture de gaz,
- et des moyens de commande pneumatiques (7, 8) commandant pneumatiquement lesdites première, deuxième, troisième et quatrième valves pneumatiques (11, 12, 13, 14).

2. Appareil selon la revendication 1, **caractérisé en ce qu'**il comprend des moyens de pilotage (16) commandant la turbine (1) pour délivrer du gaz pendant au moins des phases d'insufflation et d'exsufflation, de préférence les moyens de pilotage comprennent un contrôleur numérique relié électriquement à la turbine (1).

3. Appareil selon l'une des revendications 1 ou 2, **caractérisé en ce que** les moyens de commande pneumatiques (7, 8) commandent pneumatiquement lesdites première, deuxième, troisième et quatrième valves pneumatiques (11, 12, 13, 14) en fonction des phases d'insufflation et d'exsufflation de gaz (10) de la turbine (1), et éventuellement en fonction d'une phase transitoire séparant une phase d'insufflation d'une phase exsufflation, et/ou d'une phase de pause séparant une phase d'exsufflation d'une phase d'insufflation suivante.

4. Appareil selon l'une des revendications 1 à 3, **caractérisé en ce que**, pendant une phase d'insufflation de gaz, les moyens de commande pneumatiques (7, 8) commandent pneumatiquement :
- la deuxième valve pneumatique (12) pour empêcher toute communication fluidique entre la ligne d'insufflation (4) et l'atmosphère,
- la troisième valve pneumatique (13) pour opérer une mise en communication fluidique de la ligne d'insufflation (4) avec la ligne commune (6) de fourniture de gaz,
- la première valve pneumatique (11) pour opérer une mise en communication fluidique de la ligne d'exsufflation (5) avec l'atmosphère, et
- la quatrième valve pneumatique (14) pour empêcher toute communication fluidique entre la ligne commune (6) de fourniture de gaz et la ligne d'exsufflation (5).

5. Appareil selon l'une des revendications 1 à 3, **caractérisé en ce que**, pendant une phase d'exsufflation de gaz, les moyens de commande pneumatiques (7, 8) commandent pneumatiquement :
- la deuxième valve pneumatique (12) pour opérer une mise en communication fluidique de la ligne d'insufflation (4) avec l'atmosphère,
- la troisième valve pneumatique (13) pour empêcher toute communication fluidique entre la ligne d'insufflation (4) et la ligne commune (6) de fourniture de gaz,
- la première valve pneumatique (11) pour empêcher toute communication fluidique entre la ligne d'exsufflation (5) et l'atmosphère, et
- la quatrième valve pneumatique (14) pour opérer une mise en communication fluidique de la ligne commune (6) de fourniture de gaz avec la ligne d'exsufflation (5).

6. Appareil selon l'une des revendications précédentes, **caractérisé en ce que** les moyens de commande pneumatiques (7, 8) comprennent des première (7) et seconde (8) électrovannes pneumatiques.

7. Appareil selon la revendication 6, **caractérisé en ce que** les moyens de commande pneumatiques (7, 8) comprennent des première et seconde électrovannes pneumatiques (7, 8) de type « tout ou rien » et/ou à 3 voies.

8. Appareil selon la revendication 2 et l'une des revendications 6 ou 7, **caractérisé en ce que** les première et seconde électrovannes pneumatiques (7, 8) sont pilotées par les moyens de pilotage (16).

9. Appareil selon l'une des revendications 6 à 8, **caractérisé en ce que** les première et seconde électrovannes pneumatiques (7, 8) sont reliées fluidiquement à une source de pression positive (9) et à une source de pression négative.

10. Appareil selon la revendication 9, **caractérisé en ce que** la source de pression positive (9) comprend une pompe ou un compresseur (9) ou la source de pression négative comprend la ligne d'exsufflation (5) ou l'atmosphère ambiante.

11. Appareil selon la revendication 9, **caractérisé en ce qu'**il comprend une carcasse externe rigide (23) dans laquelle sont agencés au moins la turbine motorisée (1), la ligne d'insufflation (4), la ligne d'exsufflation (5), la ligne commune (6), les première, deuxième, troisième et quatrième valves pneumatiques (11-14), et les moyens de commande pneumatiques commandant pneumatiquement les valves pneumatiques (11-14), en particulier les électrovannes pneumatiques.

12. Appareil selon l'une des revendications 6 à 8, **caractérisé en ce que** :
- la première électrovanne pneumatique (7) commande les deuxième et quatrième valves pneumatiques (12, 14), et
- la seconde électrovanne pneumatique (8) commande les première et troisième valves pneumatiques (11, 13).

13. Appareil selon l'une des revendications précédentes, **caractérisé en ce que**, pendant une phase transitoire, les moyens de commande pneumatiques commandent pneumatiquement :
· la deuxième valve pneumatique pour empêcher toute mise en communication fluidique de la ligne d'insufflation avec l'atmosphère,
· la troisième valve pneumatique pour empêcher toute communication fluidique entre la ligne d'insufflation et la ligne commune de fourniture de gaz,
· la première valve pneumatique pour empêcher toute communication fluidique entre la ligne d'exsufflation et l'atmosphère, et
· la quatrième valve pneumatique pour empêcher toute mise en communication fluidique de la ligne commune de fourniture de gaz avec la ligne d'exsufflation.

14. Appareil selon les revendications 2 et 6, **caractérisé en ce que**, pendant une phase de pause située entre une phase d'exsufflation et la phase d'insufflation suivante, les moyens de pilotage (16) commandent les électrovannes (7, 8) et la turbine (1) pour délivrer une pression de pause supérieure ou égale à 0 mbar dans la ligne d'insufflation (4) et la ligne commune (6), de préférence une pression de pause entre 0 et 30 mbar, préférentiellement la phase de pause a une durée de pause comprise entre 0 et 5 secondes environ.

15. Appareil selon les revendications 2 et 6, **caractérisé en ce qu'**il comprend une station périphérique (30) et un module de ventilation extractible (31), le module de ventilation (31) extractible venant se loger, de manière extractible, dans la station périphérique (30), ledit module de ventilation (31) comprenant au moins la turbine (1) motorisée, la ligne d'insufflation (4), la ligne d'exsufflation (5), la ligne commune (6), les première, deuxième, troisième et quatrième valves pneumatiques (11-14), les électrovannes pneumatiques (7, 8) et les moyens de pilotage (6), de préférence le module de ventilation extractible (31) comprend la carcasse externe rigide (23).

## Patentansprüche

1. Vorrichtung (10) zur Insufflation und Exsufflation von Gas, umfassend:
- eine motorbetriebene Turbine (1), die einen Gaseinlass (2) und einen Gasauslass (3) umfasst, und
- eine Insufflationsleitung (4), die fluidisch mit dem Gasauslass (3) der Turbine (1) verbunden ist,
- eine Exsufflationsleitung (5), die fluidisch mit dem Gaseinlass (2) der Turbine (1) verbunden ist, und
- eine gemeinsame Leitung (6) zur Versorgung eines Patienten mit Gas, die fluidisch mit der Insufflationsleitung (4) und der Exsufflationsleitung (5) verbunden ist,
**dadurch gekennzeichnet, dass** sie ferner Folgendes umfasst:
- ein erstes (11) und ein viertes (14) pneumatisches Ventil, die an der Exsufflationsleitung (5) angeordnet sind, wobei das erste pneumatische Ventil (11) eine fluidische Verbindung der Exsufflationsleitung (5) mit der Atmosphäre steuert und das vierte pneumatische Ventil (14) eine fluidische Verbindung der gemeinsamen Gasversorgungsleitung (6) mit der Exsufflationsleitung (5) steuert,
- ein zweites (12) und ein drittes (13) pneumatisches Ventil, die an der Insufflationsleitung (4) angeordnet sind, wobei das zweite pneumatische Ventil (12) eine fluidische Verbindung der Insufflationsleitung (4) mit der Atmosphäre steuert und das dritte pneumatische Ventil (13) eine fluidische Verbindung der Insufflationsleitung (4) mit der gemeinsamen Gasversorgungsleitung (6) steuert,
- und pneumatische Steuermittel (7, 8), die das erste, zweite, dritte und vierte pneumatische Ventil (11, 12, 13, 14) pneumatisch steuern.

2. Vorrichtung nach Anspruch 1, **dadurch gekennzeichnet, dass** sie Ansteuermittel (16) umfasst, die die Turbine (1) ansteuern, um zumindest während Insufflations- und Exsufflationsphasen Gas abzugeben, wobei die Ansteuermittel vorzugsweise einen digitalen Controller umfassen, der elektrisch mit der Turbine (1) verbunden ist.

3. Vorrichtung nach einem der Ansprüche 1 oder 2, **dadurch gekennzeichnet, dass** die pneumatischen Steuermittel (7, 8) das erste, zweite, dritte und vierte pneumatische Ventil (11, 12, 13, 14) in Abhängigkeit von den Insufflations- und Exsufflationsphasen von Gas (10) der Turbine (1) und gegebenenfalls in Abhängigkeit von einer Übergangsphase, die eine Insufflationsphase von einer Exsufflationsphase trennt, und/oder einer Pausenphase, die eine Exsufflationsphase von einer darauffolgenden Insufflationsphase trennt, pneumatisch steuern.

4. Vorrichtung nach einem der Ansprüche 1 bis 3, **dadurch gekennzeichnet, dass** die pneumatischen Steuermittel (7, 8) während einer Gasinsufflationsphase Folgendes pneumatisch steuern:
- das zweite pneumatische Ventil (12), um eine fluidische Verbindung zwischen der Insufflationsleitung (4) und der Atmosphäre zu verhindern,
- das dritte pneumatische Ventil (13), um eine fluidische Verbindung der Insufflationsleitung (4) mit der gemeinsamen Gasversorgungsleitung (6) herzustellen,
- das erste pneumatische Ventil (11), um eine fluidische Verbindung der Exsufflationsleitung (5) mit der Atmosphäre herzustellen, und
- das vierte pneumatische Ventil (14), um eine fluidische Verbindung zwischen der gemeinsamen Gasversorgungsleitung (6) und der Exsufflationsleitung (5) zu verhindern.

5. Vorrichtung nach einem der Ansprüche 1 bis 3, **dadurch gekennzeichnet, dass** die pneumatischen Steuermittel (7, 8) während einer Gasexsufflationsphase Folgendes pneumatisch steuern:
- das zweite pneumatische Ventil (12), um eine fluidische Verbindung der Insufflationsleitung (4) mit der Atmosphäre herzustellen,
- das dritte pneumatische Ventil (13), um eine fluidische Verbindung zwischen der Insufflationsleitung (4) und der gemeinsamen Gasversorgungsleitung (6) herzustellen,
- das erste pneumatische Ventil (11), um eine fluidische Verbindung zwischen der Exsufflationsleitung (5) und der Atmosphäre zu verhindern, und
- das vierte pneumatische Ventil (14), um eine fluidische Verbindung der gemeinsamen Gasversorgungsleitung (6) mit der Exsufflationsleitung (5) herzustellen.

6. Vorrichtung nach einem der vorangehenden Ansprüche, **dadurch gekennzeichnet, dass** die pneumatischen Steuermittel (7, 8) ein erstes (7) und ein zweites (8) pneumatisches Magnetventil umfassen.

7. Vorrichtung nach Anspruch 6, **dadurch gekennzeichnet, dass** die pneumatischen Steuermittel (7, 8) ein erstes und ein zweites pneumatisches Magnetventil (7, 8) vom Typ Absperrventil und/oder Dreiwegeventil umfassen.

8. Vorrichtung nach Anspruch 2 und einem der Ansprüche 6 oder 7, **dadurch gekennzeichnet, dass** das erste und das zweite pneumatische Magnetventil (7, 8) durch die Ansteuermittel (16) gesteuert werden.

9. Vorrichtung nach einem der Ansprüche 6 bis 8, **dadurch gekennzeichnet, dass** das erste und das zweite pneumatische Elektroventil (7, 8) fluidisch mit einer Überdruckquelle (9) und mit einer Unterdruckquelle verbunden sind.

10. Vorrichtung nach Anspruch 9, **dadurch gekennzeichnet, dass** die Überdruckquelle (9) eine Pumpe oder einen Kompressor (9) umfasst oder die Unterdruckquelle die Exsufflationsleitung (5) oder die Umgebungsatmosphäre umfasst.

11. Vorrichtung nach Anspruch 9, **dadurch gekennzeichnet, dass** sie ein starres äußeres Gehäuse (23) umfasst, in dem zumindest die motorbetriebene Turbine (1), die Insufflationsleitung (4), die Exsufflationsleitung (5), die gemeinsame Leitung (6), das erste, zweite, dritte und vierte pneumatische Ventil (11 - 14) und die pneumatischen Steuermittel zum pneumatischen Steuern der pneumatischen Ventile (11 - 14), insbesondere der pneumatischen Magnetventile, angeordnet sind.

12. Vorrichtung nach einem der Ansprüche 6 bis 8, **dadurch gekennzeichnet, dass**:
- das erste pneumatische Magnetventil (7) das zweite und das vierte pneumatische Ventil (12, 14) steuert und
- das zweite pneumatische Magnetventil (8) das erste und das dritte pneumatische Ventil (11, 13) steuert.

13. Vorrichtung nach einem der vorangehenden Ansprüche, **dadurch gekennzeichnet, dass** die pneumatischen Steuermittel während einer Übergangsphase Folgendes pneumatisch steuern:
· das zweite pneumatische Ventil, um eine fluidische Verbindung der Insufflationsleitung mit der Atmosphäre zu verhindern,
· das dritte pneumatische Ventil, um eine fluidische Verbindung zwischen der Insufflationsleitung und der gemeinsamen Gasversorgungsleitung zu verhindern,
· das erste pneumatische Ventil, um eine fluidische Verbindung zwischen der Exsufflationsleitung und der Atmosphäre zu verhindern, und
· das vierte pneumatische Ventil, um eine fluidische Verbindung der gemeinsamen Gasversorgungsleitung mit der Exsufflationsleitung zu verhindern.

14. Vorrichtung nach Anspruch 2 und 6, **dadurch gekennzeichnet, dass** die Ansteuermittel (16) während der Pause zwischen einer Exsufflationsphase und der darauffolgenden Insufflationsphase die Magnetventile (7, 8) und die Turbine (1) steuern, um in der Insufflationsleitung (4) und in der gemeinsamen Leitung (6) einen Pausendruck von 0 mbar oder mehr, vorzugsweise einen Pausendruck zwischen 0 und 30 mbar, bereitzustellen, wobei die Pausenphase vorzugsweise eine Pausendauer zwischen etwa 0 und 5 Sekunden aufweist.

15. Vorrichtung nach Anspruch 2 und 6, **dadurch gekennzeichnet, dass** sie eine periphere Station (30) und ein herausziehbares Beatmungsmodul (31) umfasst, wobei das herausziehbare Beatmungsmodul (31) herausziehbar in der peripheren Station (30) untergebracht ist, wobei das Beatmungsmodul (31) zumindest die motorbetriebene Turbine (1), die Insufflationsleitung (4), die Exsufflationsleitung (5), die gemeinsame Leitung (6), das erste, zweite, dritte und vierte pneumatische Ventil (11 - 14), die pneumatischen Elektroventile (7, 8) und die Ansteuermittel (6) umfasst, wobei das herausziehbare Beatmungsmodul (31) vorzugsweise das äußere Gehäuse (23) umfasst.

## Claims

1. Gas insufflation and exsufflation apparatus (10) comprising:
- a motorized turbine (1) comprising a gas inlet (2) and a gas outlet (3), and
- an insufflation line (4) fluidically connected to the gas outlet (3) of the turbine (1),
- an exsufflation line (5) fluidically connected to the gas inlet (2) of the turbine (1), and
- a common line (6) for supplying gas to a patient, which is fluidically connected to said insufflation line (4) and exsufflation line (5),
**characterized in that** it further comprises:
- a first (11) and a fourth (14) pneumatic valve which are arranged on the exsufflation line (5), the first pneumatic valve (11) controlling an establishment of fluidic communication of the exsufflation line (5) with the atmosphere, and the fourth pneumatic valve (14) controlling an establishment of fluidic communication of the common gas supply line (6) with the exsufflation line (5),
- a second (12) and a third (13) pneumatic valve which are arranged on the insufflation line (4), the second pneumatic valve (12) controlling an establishment of fluidic communication of the insufflation line (4) with the atmosphere, and the third pneumatic valve (13) controlling an establishment of fluidic communication of the insufflation line (4) with the common gas supply line (6),
- and pneumatic control means (7, 8) pneumatically controlling said first, second, third and fourth pneumatic valves (11, 12, 13, 14).

2. Apparatus according to Claim 1, **characterized in that** it comprises control means (16) controlling the turbine (1) to deliver gas during at least insufflation and exsufflation phases, the control means preferably comprising a digital controller connected electrically to the turbine (1).

3. Apparatus according to either of Claims 1 and 2, **characterized in that** the pneumatic control means (7, 8) pneumatically control said first, second, third and fourth pneumatic valves (11, 12, 13, 14) according to the gas insufflation and exsufflation phases (10) of the turbine (1), and possibly according to a transient phase separating an insufflation phase from an exsufflation phase, and/or according to a pause phase separating an exsufflation phase from a following insufflation phase.

4. Apparatus according to one of Claims 1 to 3, **characterized in that**, during a gas insufflation phase, the pneumatic control means (7, 8) pneumatically control:
- the second pneumatic valve (12) to prevent any fluidic communication between the insufflation line (4) and the atmosphere,
- the third pneumatic valve (13) to bring the insufflation line (4) into fluidic communication with the common gas supply line (6),
- the first pneumatic valve (11) to bring the exsufflation line (5) into fluidic communication with the atmosphere, and
- the fourth pneumatic valve (14) to prevent any fluidic communication between the common gas supply line (6) and the exsufflation line (5).

5. Apparatus according to one of Claims 1 to 3, **characterized in that**, during a gas exsufflation phase, the pneumatic control means (7, 8) pneumatically control:
- the second pneumatic valve (12) to bring the insufflation line (4) into fluidic communication with the atmosphere,
- the third pneumatic valve (13) to prevent any fluidic communication between the insufflation line (4) and the common gas supply line (6),
- the first pneumatic valve (11) to prevent any fluidic communication between the exsufflation line (5) and the atmosphere, and
- the fourth pneumatic valve (14) to bring the common gas supply line (6) into fluidic communication with the exsufflation line (5).

6. Apparatus according to one of the preceding claims, **characterized in that** the pneumatic control means (7, 8) comprise first (7) and second (8) pneumatic solenoid valves.

7. Apparatus according to Claim 6, **characterized in that** the pneumatic control means (7, 8) comprise first and second pneumatic solenoid valves (7, 8) of the "all or nothing" and/or 3-way type.

8. Apparatus according to Claim 2 and either of Claims 6 and 7, **characterized in that** the first and second pneumatic solenoid valves (7, 8) are controlled by the control means (16).

9. Apparatus according to one of Claims 6 to 8, **characterized in that** the first and second pneumatic solenoid valves (7, 8) are fluidically connected to a positive pressure source (9) and to a negative pressure source.

10. Apparatus according to Claim 9, **characterized in that** the positive pressure source (9) comprises a pump or a compressor (9) or the negative pressure source comprises the exsufflation line (5) or the ambient atmosphere.

11. Apparatus according to Claim 9, **characterized in that** it comprises a rigid outer shell (23) in which are arranged at least the motorized turbine (1), the insufflation line (4), the exsufflation line (5), the common line (6), the first, second, third and fourth pneumatic valves (11-14), and the pneumatic control means pneumatically controlling the pneumatic valves (11-14), in particular the pneumatic solenoid valves.

12. Apparatus according to one of Claims 6 to 8, **characterized in that**:
- the first pneumatic solenoid valve (7) controls the second and fourth pneumatic valves (12, 14), and
- the second pneumatic solenoid valve (8) controls the first and third pneumatic valves (11, 13).

13. Apparatus according to one of the preceding claims, **characterized in that**, during a transient phase, the pneumatic control means pneumatically control:
• the second pneumatic valve to prevent any establishment of fluidic communication of the insufflation line with the atmosphere,
• the third pneumatic valve to prevent any fluidic communication between the insufflation line and the common gas supply line,
• the first pneumatic valve to prevent any fluidic communication between the exsufflation line and the atmosphere, and
• the fourth pneumatic valve to prevent any establishment of fluidic communication of the common gas supply line with the exsufflation line.

14. Apparatus according to Claims 2 and 6, **characterized in that**, during a pause phase situated between an exsufflation phase and the following insufflation phase, the control means (16) control the solenoid valves (7, 8) and the turbine (1) to deliver a pause pressure greater than or equal to 0 mbar in the insufflation line (4) and the common line (6), preferably a pause pressure between 0 and 30 mbar, the pause phase preferably having a pause duration of between 0 and 5 seconds approximately.

15. Apparatus according to Claims 2 and 6, **characterized in that** it comprises a peripheral station (30) and an extractable ventilation module (31), the extractable ventilation module (31) being housed, in an extractable manner, in the peripheral station (30), said ventilation module (31) comprising at least the motorized turbine (1), the insufflation line (4), the exsufflation line (5), the common line (6), the first, second, third and fourth pneumatic valves (11-14), the pneumatic solenoid valves (7, 8) and the control means (6), the extractable ventilation module (31) preferably comprising the rigid outer shell (23).
